# EUROPEAN PATENT APPLICATION

(11) **EP 4 349 851 A1**
(43) Date of publication of application: **10.04.2024**
(21) Application number: 22815338.3
(22) Date of filing: 02.06.2022
(51) Int. Cl.: C07K 7/56, A61K 38/12, A61P 31/10

(54) **METHOD FOR PREPARING ANIDULAFUNGIN DERIVATIVE**

(30) Priority: 03.06.2021 CN 202110617888
(71) Applicant: Shanghai Senhui Medicine Co., Ltd., Shanghai 201203 (CN); Shanghai Shengdi Pharmaceutical Co., Ltd, Shanghai 201210 (CN); Jiangsu Hengrui Pharmaceuticals Co., Ltd., Lianyungang, Jiangsu 222047 (CN)
(72) Inventor: JIANG, Wei, Shanghai 201203 (CN); HUANG, Jian, Shanghai 201203 (CN); HU, Junqiang, Shanghai 201203 (CN); CAO, Weicou, Shanghai 201203 (CN); ZHOU, Yinan, Shanghai 201203 (CN)
(74) Representative: Potter Clarkson
(86) International application number: PCT/CN2022/096760
(87) International publication number: WO 2022/253297

(57) **Abstract**

The present disclosure relates to a method for preparing an anidulafungin derivative. In particular, the present disclosure relates to a method for preparing an anidulafungin derivative as represented by formula (I) and an intermediate thereof.

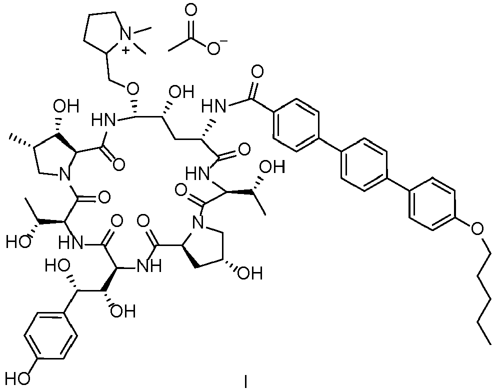

## Description

### TECHNICAL FIELD

The present disclosure belongs to the field of pharmaceutical chemistry, relates to a method for preparing an anidulafungin derivative, and particularly provides a preparation method for a compound of formula (I).

### BACKGROUND

The development of antifungal treatment regimens has been a consistent challenge for today's society. Currently available drugs for the treatment of fungal infections include amphotericin B, a macrolide polyene that interacts with fungal membrane sterols; flucytosine, a fluoropyrimidine that interacts with fungal protein and DNA biosynthesis; and a variety of azole antifungals that inhibit fungal membrane-sterol biosynthesis (e.g., ketoconazole, itraconazole and fluconazole) (Alexander et al., Drugs, 54:657, 1997). The application of amphotericin B is restricted for the infusion-related reactions and renal toxicity, in spite of the fact that it has a wide range of activity and is considered the "gold standard" for antifungal therapy (Warnock, J. Antimicrob. Chemother., 41:95, 1998). The use of flucytosine is also restricted due to the development of drug-resistant microorganisms and its narrow spectrum of activity. The widespread use of azole antifungals is leading to the occurrence of clinical drug-resistant strains of *Candida spp.* Echinocandins are a novel class of antifungals. They generally comprise a cyclic hexapeptide and a lipophilic tail, with the latter linked to the hexapeptide core by an amide bond. Such drugs interfere with the synthesis of β-1,3-glucose in fungal cell walls by non-competitive inhibition of β-1,3-glucose synthase, leading to changes of the fungal cell walls in permeability and to lysis, and thus death, of the cells. Due to the absence of cell walls in human cells and the presence of cell walls in fungal cells, echinocandin antifungals can directly act upon the components of the fungal cell walls, thereby having low toxicity to humans. Therefore, they have been one of the safest antifungals to date.

Currently, such drugs on the market include caspofungin, micafungin and anidulafungin. Caspofungin, the first echinocandin antifungal, was developed by Merck Sharp & Dohme, U.S. and was approved by USFDA for the treatment of fungal infections in 2004 and approved for the treatment of *Candida* infections in children in 2008. Micafungin (Mycamine) is a novel semi-synthetic antifungal, launched in Japan in 2002. Anidulafungin is a third-generation semi-synthetic echinocandin antifungal, launched in 2006.

The application PCT/CN2020/133815 provides an anidulafungin derivative of formula (I). The compound has been found to have relatively strong antifungal activity.

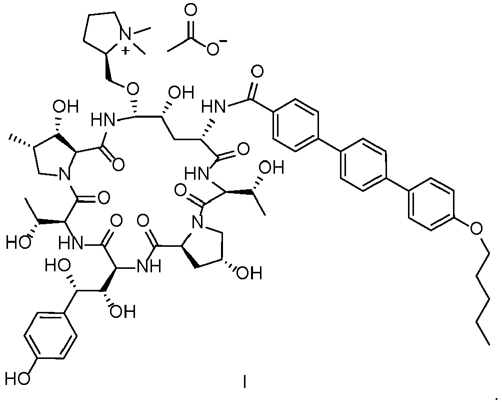

### SUMMARY

The present disclosure provides a method for preparing a compound of formula (I), comprising the following step: reacting anidulafungin with a compound of formula (II) to give a product,

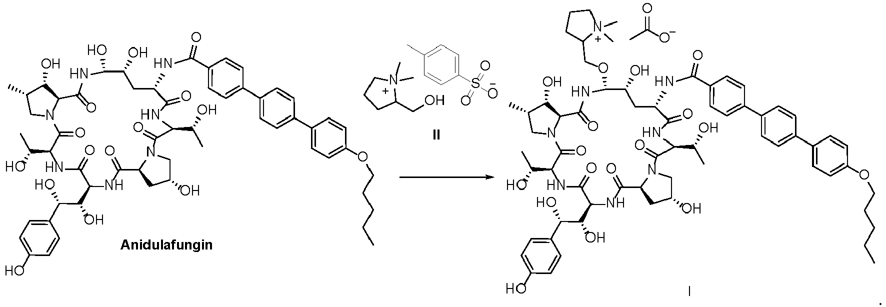

In some embodiments, the reaction is carried out in the presence of acids A1 and A2.

In some embodiments, the acid A1 is phenylboronic acid or 3,4-dimethoxyphenylboronic acid.

In some embodiments, the acid A1 is 3,4-dimethoxyphenylboronic acid.

In some embodiments, the acid A2 is p-toluenesulfonic acid, camphorsulfonic acid or trifluoroacetic acid.

In some embodiments, the acid A2 is camphorsulfonic acid or trifluoroacetic acid.

In some embodiments, the acid A2 is trifluoroacetic acid.

In some embodiments, the reaction is carried out in a solvent S1.

In some embodiments, the solvent S1 is anhydrous dioxane or acetonitrile.

In some embodiments, the solvent S1 is acetonitrile.

In some embodiments, the molar ratio of anidulafungin to the compound of formula (II) is 1:(1-50).

In some embodiments, the molar ratio of anidulafungin to the compound of formula (II) is 1:(1-30).

In some embodiments, the molar ratio of anidulafungin to the compound of formula (II) is 1:(1-10).

In some embodiments, the molar ratio of anidulafungin to the compound of formula (II) is 1:(1-6).

In some embodiments, the molar ratio of anidulafungin to the compound of formula (II) is 1:30.

In some embodiments, the molar ratio of anidulafungin to the compound of formula (II) is 1:6.

In some embodiments, the molar ratio of anidulafungin to the acid A1 is 1:(1-10).

In some embodiments, the molar ratio of anidulafungin to the acid A1 is 1:(1-5).

In some embodiments, the molar ratio of anidulafungin to the acid A1 is 1:(1-2).

In some embodiments, the molar ratio of anidulafungin to the acid A1 is 1:1.3.

In some embodiments, the molar ratio of anidulafungin to the acid A2 is 1:(1-10).

In some embodiments, the molar ratio of anidulafungin to the acid A2 is 1:(1-5).

In some embodiments, the molar ratio of anidulafungin to the acid A2 is 1:5.

In some embodiments, the weight-to-volume ratio of anidulafungin to the acid A2, measured in g/mL, is 1:(1-5).

In some embodiments, the weight-to-volume ratio of anidulafungin to the acid A2, measured in g/mL, is 1:(1-2.5).

In some embodiments, the weight-to-volume ratio of anidulafungin to the acid A2, measured in g/mL, is 1:2.5.

In some embodiments, the reaction temperature is 0-50 °C.

In some embodiments, the reaction temperature is 10-40 °C.

In some embodiments, the reaction temperature is 20-30 °C.

In some embodiments, the reaction temperature is room temperature.

In some embodiments, the specific steps of the reaction are: dissolving anidulafungin and the acid A1 in the solvent S2, stirring at room temperature, concentrating to dryness, adding the compound of formula (II), the acid A2 and the solvent S1, stirring at room temperature in a nitrogen atmosphere, adding an aqueous solution of sodium acetate to quench the reaction, concentrating to give a crude product, and purifying by HPLC to give the product.

In some embodiments, the solvent S2 is THF.

In some embodiments, the specific steps of the reaction are: dissolving 1 eq of anidulafungin and 2 eq of phenylboronic acid in THF, stirring at room temperature for 1 h, concentrating to dryness, adding 6 eq of the compound of formula (II), 5 eq of camphorsulfonic acid, and anhydrous dioxane, stirring at room temperature overnight in a nitrogen atmosphere, adding an aqueous solution of sodium acetate to quench the reaction, concentrating to give a crude product, and purifying by HPLC to give the product.

In some embodiments, the specific steps of the reaction are: dissolving 1 eq of anidulafungin and 1.3 eq of 3,4-dimethoxyphenylboronic acid in THF, stirring at room temperature for 1 h, concentrating to dryness, adding 30 eq of the compound of formula (II), trifluoroacetic acid at a volume-to-weight ratio of 2.5, and acetonitrile, stirring at room temperature for 3 h in a nitrogen atmosphere, adding an aqueous solution of sodium acetate to quench the reaction, concentrating to give a crude product, and purifying by HPLC to give the product; wherein the volume-to-weight ratio is the volume-to-weight ratio of trifluoroacetic acid to anidulafungin, measured in mL/g.

In some embodiments, the preparation method further comprises the following step: reacting N-methylprolinol with methyl p-toluenesulfonate to give a product,

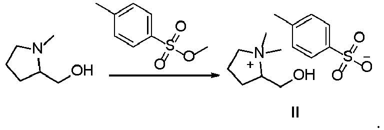

In some embodiments, the molar ratio of N-methylprolinol to methyl p-toluenesulfonate is 1:(1-5).

In some embodiments, the molar ratio of N-methylprolinol to methyl p-toluenesulfonate is 1:(1-3).

In some embodiments, the molar ratio of N-methylprolinol to methyl p-toluenesulfonate is 1:1.

In some embodiments, the reaction for preparing the compound of formula (II) is carried out in a solvent S3.

In some embodiments, the solvent S3 is acetone.

In some embodiments, the temperature of the reaction for preparing the compound of formula (II) is 0-80 °C.

In some embodiments, the temperature of the reaction for preparing the compound of formula (II) is 10-70 °C.

In some embodiments, the temperature of the reaction for preparing the compound of formula (II) is 20-60 °C.

In some embodiments, the temperature of the reaction for preparing the compound of formula (II) is 30-60 °C.

In some embodiments, the temperature of the reaction for preparing the compound of formula (II) is 40-60 °C.

In some embodiments, the temperature of the reaction for preparing the compound of formula (II) is 50-60 °C.

In some embodiments, the specific steps of the reaction are: dissolving N-methylprolinol in the solvent S3, slowly adding methyl p-toluenesulfonate, heating and stirring the reaction, adding petroleum ether to precipitate a solid, filtering and drying to give the product.

In some embodiments, the specific steps of the reaction are: dissolving N-methylprolinol in acetone, slowly adding methyl p-toluenesulfonate, heating at reflux and stirring the reaction, adding petroleum ether to precipitate a solid, filtering and drying to give the product.

In some embodiments, the specific steps of the reaction are: dissolving 1 eq of N-methylprolinol in acetone, slowly adding 1 eq of methyl p-toluenesulfonate, heating at reflux and stirring the reaction, adding petroleum ether to precipitate a solid, filtering and drying to give the product.

In some embodiments, the method comprises the following steps: dissolving 1 eq of N-methylprolinol in acetone, slowly adding 1 eq of methyl p-toluenesulfonate, heating at reflux and stirring the reaction, adding petroleum ether to precipitate a solid, filtering and drying to give the compound of formula (II);
dissolving 1 eq of anidulafungin and 2 eq of phenylboronic acid in THF, stirring at room temperature for 1 h, concentrating to dryness, adding 6 eq of the compound of formula (II), 5 eq of camphorsulfonic acid, and anhydrous dioxane, stirring at room temperature overnight in a nitrogen atmosphere, adding an aqueous solution of sodium acetate to quench the reaction, concentrating to give a crude product, and purifying by HPLC to give the anidulafungin derivative of formula (I).

In some embodiments, the method comprises the following steps: dissolving 1 eq of N-methylprolinol in acetone, slowly adding 1 eq of methyl p-toluenesulfonate, heating at reflux and stirring the reaction, adding petroleum ether to precipitate a solid, filtering and drying to give the compound of formula (II);
dissolving 1 eq of anidulafungin and 1.3 eq of 3,4-dimethoxyphenylboronic acid in THF, stirring at room temperature for 1 h, concentrating to dryness, adding 30 eq of the compound of formula (II), trifluoroacetic acid at a volume-to-weight ratio of 2.5, and acetonitrile, stirring at room temperature for 3 h in a nitrogen atmosphere, adding an aqueous solution of sodium acetate to quench the reaction, concentrating to give a crude product, and purifying by HPLC to give the anidulafungin derivative of formula (I); wherein the volume-to-weight ratio is the volume-to-weight ratio of trifluoroacetic acid to anidulafungin, measured in mL/g.

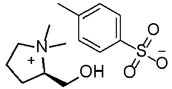

In some embodiments, the compound of formula (II) is , and the compound of formula (I) is

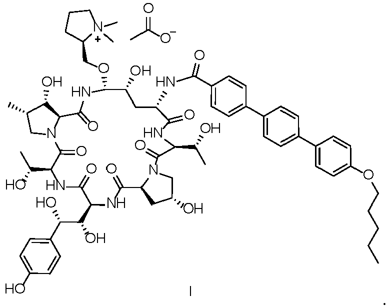

The present disclosure also provides an intermediate compound of formula (II):

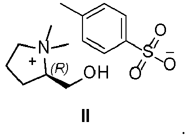

### BRIEF DESCRIPTION OF THE DRAWINGS

FIG. 1 shows changes in histamine concentration after intravenous administration of compounds.
FIG. 2 shows a comparison of the histamine concentrations at 30 min after intravenous administration of compounds.

### DETAILED DESCRIPTION

The present invention is further described below with reference to examples, but these examples are not intended to limit the scope of the present invention.

Experimental procedures without specific conditions indicated in the following examples, are generally conducted according to conventional conditions, or according to conditions recommended by the manufacturers of the starting materials or commercial products. Reagents without specific origins indicated are commercially available conventional reagents.

Anidulafungin and caspofungin were both purchased from Taizhou KEDE Chemical. Rezafungin was synthesized according to CN103889221A.

HPLC purity analysis method: chromatography column: Welch Xtimate C18 (3 µm,4.6 mm × 150 mm), mobile phase: 0.05% TFA in water/0.05% TFA in ACN, detection wavelength: UV 214 nm.

The structures of compounds were determined by nuclear magnetic resonance (NMR) spectroscopy and/or mass spectrometry (MS). NMR shifts (δ) are given in 10⁻⁶ (ppm). NMR analysis was performed using a Bruker AVANCE-400 nuclear magnetic resonance instrument, with deuterated dimethyl sulfoxide (DMSO-*d₆*), deuterated chloroform (CDCl₃) and deuterated methanol (CD₃OD) as solvents and tetramethylsilane (TMS) as an internal standard.

The monitoring of the reaction progress in the examples was conducted by thin-layer chromatography (TLC). The developing solvent for reactions, the eluent system of column chromatography for compound purification and the developing solvent system of thin-layer chromatography include: A: dichloromethane/methanol system, B: n-hexane/ethyl acetate system, C: petroleum ether/ethyl acetate system, and D: petroleum ether/ethyl acetate/methanol system. The volume ratio of the solvents was adjusted according to the polarity of the compound, or by adding a small amount of basic or acidic reagents such as triethylamine and acetic acid.

High-resolution mass spectrometry: chromatography column: Waters BEH C18 1.7U 2.1 × 50 mm, mobile phase: 0.1% FA in water/0.1% FA in acetonitrile.

Room temperature refers to 25 °C.

### Example 1

### Step 1

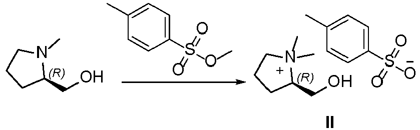

N-Methyl-D-prolinol (3.00 g, 26.05 mmol) was dissolved in 30 mL of acetone, and methyl p-toluenesulfonate (4.85 g, 1.0 eq.) was slowly added. The mixture was heated at reflux and stirred for 4 h, and petroleum ether was added to precipitate a solid. After filtration and drying, the compound of formula (II) was obtained as a white solid (7.33 g, 93.4% yield). Ms: 130.0[M⁺].

¹H NMR (400 MHz, MeOD-d₄) δ 7.73 (d, *J* = 8.00 Hz, 2H), 7.27 (d, J = 8.00Hz, 2H), 3.96-3.93 (m, 1H), 3.84-3.73 (m, 2H), 3.63-3.56 (m, 2H), 3.27 (s, 3H), 3.05 (s, 3H), 2.40 (s, 3H), 2.27-2.24 (m, 1H), 2.14-2.10 (m, 2H), 1.93-1.91 (m, 1H).

### Step 2

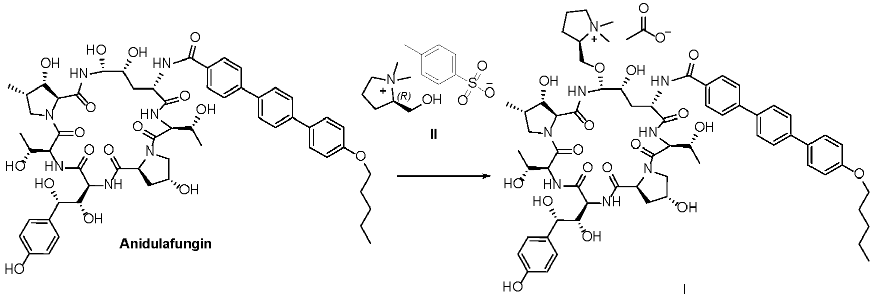

Anidulafungin (2.5 g, 2.19 mmol) and phenylboronic acid (0.535 g, 2 eq.) were dissolved in THF (50 mL). The solution was stirred at room temperature for 1 h and concentrated to dryness. The compound of formula (II) (3.96 g, 6 eq.), camphorsulfonic acid (2.54 g, 5 eq.) and anhydrous dioxane (75 mL) were added. The mixture was stirred overnight at room temperature in a nitrogen atmosphere, and an aqueous solution of sodium acetate was added to quench the reaction. The reaction mixture was concentrated to give a crude product, and the crude product was purified by preparative HPLC to give a product (the compound of formula (I), 1.96 g, 96.9% purity, 68% yield). HRMS: 1251.6173 [M⁺].

¹H NMR (400 MHz, METHANOL-d₄) δ 7.98 (d, *J* = 8.8 Hz, 2H), 7.81 (d, J = 8.0 Hz, 2H), 7.69-7.76 (m, 4H), 7.61 (d, *J* = 9.2 Hz, 2H), 7.15 (d, *J* = 8.8 Hz, 2H), 7.01 (d, *J* = 8.8 Hz, 2H), 6.76 (d, *J* = 8.4 Hz, 2H), 5.42 (d, J = 2.4 Hz, 1H), 5.03 (d, J= 3.2 Hz, 1H), 4.92-4.93 (m,1H), 4.74-4.78 (m, 1H), 4.57-4.61 (m, 3H), 4.38 (d, J = 4.0 Hz, 1H), 4.32-4.34 (m, 2H), 4.24-4.28 (m, 2H), 4.16-4.20 (m, 1H), 4.06-4.10 (m, 1H), 3.97-4.04 (m, 4H), 3.81-3.92 (m, 4H), 3.46-3.63 (m, 3H), 3.21(s, 3H), 3.00 (s, 3H), 2.42-2.52 (m, 2H), 2.26-2.31 (m, 2H), 1.92-2.15 (m, 5H), 1.90 (s, 3H), 1.78-1.85 (m, 2H), 1.40-1.52 (m, 4H), 1.25-1.28 (m, 6H), 1.08 (d, J = 6.8 Hz, 3H), 0.97 (t, *J* = 6.8 Hz, 3H).

### Example 2

### Step 1

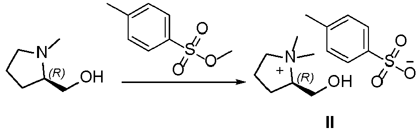

N-Methyl-D-prolinol (3.00 g, 26.05 mmol) was dissolved in 30 mL of acetone, and methyl p-toluenesulfonate (4.85 g, 1.0 eq.) was slowly added. The mixture was heated at reflux and stirred for 4 h, and petroleum ether was added to precipitate a solid. After filtration and drying, the compound of formula (II) was obtained as a white solid (7.33 g, 93.4% yield). Ms: 130.0[M⁺].

¹H NMR (400 MHz, MeOD-d₄) δ 7.73 (d, *J* = 8.00 Hz, 2H), 7.27 (d, J = 8.00 Hz, 2H), 3.96-3.93 (m, 1H), 3.84-3.73 (m, 2H), 3.63-3.56 (m, 2H), 3.27 (s, 3H), 3.05 (s, 3H), 2.40 (s, 3H), 2.27-2.24 (m, 1H), 2.14-2.10 (m, 2H), 1.93-1.91 (m, 1H).

### Step 2

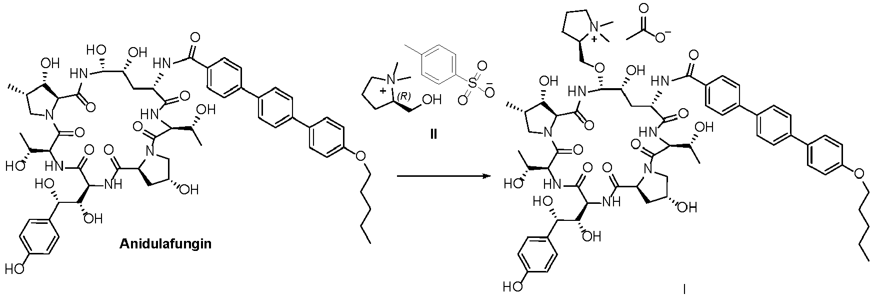

Anidulafungin (2.0 g, 1.75 mmol) and 3,4-dimethoxyphenylboronic acid (0.415 g, 1.3 eq.) were dissolved in THF (40 mL). The solution was stirred at room temperature for 1 h and concentrated to dryness. The compound of formula (II) (15.86 g, 30 eq.), trifluoroacetic acid (5 mL, 2.5 v) and acetonitrile (20 mL) were added. The mixture was stirred at room temperature for 3 h in a nitrogen atmosphere, and an aqueous solution of sodium acetate was added to quench the reaction. The reaction mixture was concentrated to give a crude product, and the crude product was purified by preparative HPLC to give a product (the compound of formula (I), 1.95 g, 98.5% purity, 85% yield). HRMS: 1251.6173 [M⁺].

¹H NMR (400 MHz, METHANOL-d₄) δ 7.98 (d, *J* = 8.8 Hz, 2H), 7.81 (d, J = 8.0 Hz, 2H), 7.69-7.76 (m, 4H), 7.61 (d, *J* = 9.2 Hz, 2H), 7.15 (d, *J* = 8.8 Hz, 2H), 7.01 (d, *J* = 8.8 Hz, 2H), 6.76 (d, *J* = 8.4 Hz, 2H), 5.42 (d, J = 2.4 Hz, 1H), 5.03 (d, J = 3.2 Hz, 1H), 4.92-4.93 (m,1H), 4.74-4.78 (m, 1H), 4.57-4.61 (m, 3H), 4.38 (d, J = 4.0 Hz, 1H), 4.32-4.34 (m, 2H), 4.24-4.28 (m, 2H), 4.16-4.20(m, 1H), 4.06-4.10 (m, 1H), 3.97-4.04 (m, 4H), 3.81-3.92 (m, 4H), 3.46-3.63 (m, 3H), 3.21(s, 3H), 3.00 (s, 3H), 2.42-2.52 (m, 2H), 2.26-2.31 (m, 2H), 1.92-2.15 (m, 5H), 1.90 (s, 3H), 1.78-1.85 (m, 2H), 1.40-1.52 (m, 4H), 1.25-1.28 (m, 6H), 1.08 (d, J = 6.8 Hz, 3H),0.97 (t, *J* = 6.8 Hz, 3H).

### Test Example 1: Test Method for Antifungal Activity

After a test compound was serially diluted, an MIC (minimum inhibitory concentration) assay was performed on the standard *Candida* strain and an MEC (minimum effective concentration) assay on the standard *Aspergillus* strain. The MIC assay was performed according to the guidelines of the Clinical and Laboratory Standards Institute (CLSI M27-A3) and the MEC assay according to the guidelines of the Clinical and Laboratory Standards Institute (CLSI M38-A2).

### Preparation of fungal inoculation liquid

### Candida:

The frozen strain was passaged at least twice, and a single colony was picked and resuspended in normal saline or sterile water in a tube. The suspension was vortexed and adjusted to 0.5 McF (1 × 10⁶ to 5 × 10⁶ CFU/mL) using a spectrophotometer at wavelength 530 nm. The suspension was 50-fold diluted with normal saline and then 20-fold diluted with 1×RPMI 1640 broth (1 × 10³ to 5 × 10³ CFU/mL). 10 µL of the suspension was applied to an SDA plate for colony counting, with a range from about 10 to 50 single colonies.

After complete dissolution was achieved at room temperature in the prepared susceptibility testing plate, the bacterial suspension was added to a 96-well plate at 100 µL per well using a multi-channel pipette. At this time, the bacterium concentration in each well should be 0.5 × 10³ to 2.5 × 10³ CFU/mL.

### Aspergillus (operation in class II biosafety cabinet):

*Aspergillus* was passaged onto an SDA plate and cultured at 35 °C for 48 h to 7 d to induce sporulation. Colonies on the plate were covered with about 1 mL of 0.85% normal saline or sterile water (polysorbate 20 was added at a final concentration of 0.1%-0.01%). The medium was gently wiped on its surface with a tip or a sterile cotton swab (be careful not to break the medium), and the spore hyphae suspension was transferred to a sterile tube and let stand for 3-5 min so that the heavy particles settled. The homogeneous upper layer of the suspension was transferred to a new sterile tube, which was then sealed and vortexed for 15 s (be careful as the suspension may produce an aerosol when the cover is removed). The concentration of the suspension was adjusted until an OD value of 0.09-0.13 was achieved using a spectrophotometer at 530 nm. The suspension was 50-fold diluted with 1×RPMI 1640. 100 µL of sample was added to each well of the 96-well plate within 2 h after dilution (the final spore concentration in the susceptibility testing plate was at 0.4 × 10⁴ to 5 × 10⁴ CFU/mL).

Colony counting: The suspension diluted with RPMI 1640 was further diluted 10-fold, and 10 µL of the dilution was applied to an SDA plate, cultured at 28 °C, and observed every day; colonies were immediately counted upon being visible to the naked eye.

### Culture

The assay plate for yeast-type fungi was incubated in an incubator at 35 °C with 85% humidity for 24 h, and then the MIC value was read. For echinocandin drugs, *Aspergillus* was incubated at 28 °C for 21-26 h, and then the MEC results were read.

### MIC or MEC interpretation

Yeast-type fungi: A disposable sealing film was applied to the 96-well plate, and the mixture was well mixed by shaking. Observation was performed through a plate reader with the naked eye. Comparisons were made to the growth control, and the minimum compound concentration corresponding to ≥50% growth inhibition was defined as MIC. Pictures were taken and saved using an automatic plate reader.

*Aspergillus*: For echinocandin drugs, comparisons were made to the growth control under a plate reader, and the minimum drug concentration that could cause the hyphae to form small, round, compact hyphal particles was defined as MEC. For accurate determination of MEC values, the plate must not be vortexed before reading.

**Table 1. Bacteriostatic activity assay results for compounds (first batch)**

| Compound | Initial assay concentration (µg/mL) | *Candida albicans* | *Candida glabrata* | *Candida parapsilosis* | *Candida krusei* | *Meyerozyma guilliermondii* | *Candida tropicalis* | *Aspergillus flavus* |
|---|---|---|---|---|---|---|---|---|
| | | ATCC 90029 | ATCC 15126 | ATCC 22019 | ATCC 6258 | ATCC 6260 | ATCC 750 | ATCC 28539 |
| Anidulafungin | 16 | 0.016 | 0.125 | 0.5 | 0.063 | 1 | 0.016 | 0.031 |
| Caspofungin acetate | 16 | 0.063 | 0.25 | 1 | 1 | 1 | 0.016 | 0.031 |
| Rezafungin acetate | 16 | 0.125 | 0.125 | 2 | 0.25 | 2 | 0.016 | 0.031 |
| Compound of formula (I) | 16 | 0.031 | / | 1 | 0.047 | 1 | 0.016 | 0.031 |

| | | | | | | | | |
|---|---|---|---|---|---|---|---|---|
| Note: 1. *Candida parapsilosis* ATCC 22019 and *Candida krusei* ATCC6258 were quality control strains. According to CLSI-M60, the 24 h MIC of ANI for ATCC 22019 is (0.25-2) µg/mL, the CAS is (0.25-1) µg/mL, the 24 h MIC of ANI for ATCC6258 is (0.03-0.12) µg/mL, and the CAS is (0.12-1) µg/mL. | | | | | | | | |

**Table 2. Bacteriostatic activity assay results for compounds (second batch)**

| Compound | Initial assay concentration (µg/mL) | *Candida albicans* | *Candida albicans* + 50% human serum | *Candida albicans* | *Candida glabrata* | *Candida albicans* (azole drug-resistant strain) | *Candida albicans* (azole drug-resistant strain) | *Candida tropicalis* (amphotericin-resistant strain) |
|---|---|---|---|---|---|---|---|---|
| | | ATCC 90028 | ATCC 90028 | ATCC 44858 | ATCC 36583 | R357 | R358 | ATCC 200956 |
| Rezafungin acetate | 16 | 0.25 | 0.25 | 0.25 | 0.25 | 0.25 | 0.25 | 0.25 |
| Compound of formula (I) | 16 | 0.25 | 0.25 | 0.25 | 0.5 | 0.5 | 0.25 | 0.25 |

The assay data show that the compound of formula (I) of the present disclosure has excellent antifungal activity.

### Test Example 2: Plasma Histamine Concentrations of Compounds and Pharmacokinetic Study

### Test method:

12 SD rats were divided into 2 groups of 6 (half are male and half female). The rats were observed at least once a day. The body weights were measured once before administration. Administration was performed by single intravenous injection for 20 min per animal. PK measurement was performed once before administration and 5 min, 30 min, 1 h, 4 h, 8 h, 24 h, 48 h, 72 h and 96 h after administration. A histamine assay was performed once before administration and 30 min, 4 h, 8 h and 24 h after administration.

The dose design is shown in the table below:

| Group | Route of administration | Dose (mg/kg) | Concentration (mg/mL) | Volume (mL/kg) | Number of animals | |
|---|---|---|---|---|---|---|
| | | | | | Male | Female |
| 1 (compound of formula (I)) | Intravenous injection | 10 | 2 | 5 | 3 | 3 |
| 2 (Rezafungin acetate group) | Intravenous injection | 10 | 2 | 5 | 3 | 3 |

**The results are mainly as follows:**

### General state observation

A transient slight decrease in activity occurred in 2 females in group 2 (2/3 of the rats) on the day of administration.

Apart from that, the SD rats in each group were in good general conditions, showed normal spontaneous locomotor activity, had clean skin and hair, and showed normal defecation and urination, and no other abnormality was observed.

### Histamine assay

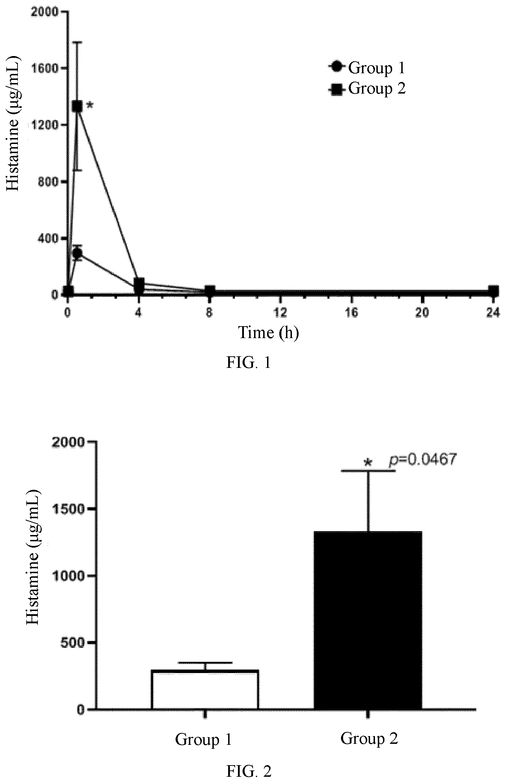

A transient increase in the histamine level was caused in both group 1 and group 2 by intravenous administration. The plasma histamine concentration peaked at 30 min, showed a tendency to recover at 4 h, and substantially returned to the normal level at 8-24 h, as shown in FIG. 1. 30 min after administration, the mean histamine concentration in the plasma of the rats in group 2 was 1333.0 ng/mL, which is 4.5 times significantly (p = 0.046) higher than that in group 1 (296.6 ng/mL), as shown in FIG. 2. The ability of the group 1 compound to induce increases in the histamine level in rats is significantly lower than that of the group 2 compound when they are administered at the same dose.

### Pharmacokinetics

The pharmacokinetic parameters in animals after administration to group 1 or group 2 are shown in the table below:

| Group | Sex | T_{1/2} | Cₘₐₓ | AUC₀₋₉₆ | AUC_{0-inf} | CL | MRT_{IV} | Vd_{SS} |
|---|---|---|---|---|---|---|---|---|
| | | h | pg/mL | hr*µg/mL | hr*µg/mL | mL/min/kg | hr | L/kg |
| 1 | Male | 27.7 | 8.0 | 173.9 | 191.2 | 0.9 | 37.1 | 1.9 |
| | Female | 25.5 | 9.3 | 179.6 | 195.0 | 0.9 | 34.6 | 1.8 |
| 2 | Male | 28.0 | 7.8 | 190.5 | 212.1 | 0.8 | 38.4 | 1.8 |
| | Female | 27.9 | 8.6 | 205.8 | 227.2 | 0.8 | 39.1 | 1.8 |

The assay data show that after administration at the same dose by single intravenous injection, group 1 and group 2 are close to each other in the plasma drug exposure levels (Cₘₐₓ and AUC) and show no significant sex-related difference, and the other pharmacokinetic parameters each have substantially equivalent values for both groups.

In conclusion, after administration at the same dose (10 mg/kg) by single intravenous injection, the plasma drug exposure levels of the compound of formula (I) and rezafungin acetate are close, while the ability of the compound of formula (I) to induce increases in the histamine level in rats is significantly lower than that of rezafungin acetate, suggesting that the compound of formula (I), when applied clinically, will not easily cause allergies compared to rezafungin.

## Claims

1. A method for preparing an anidulafungin derivative of formula (I), comprising the following step: reacting anidulafungin with a compound of formula (II) to give a product,

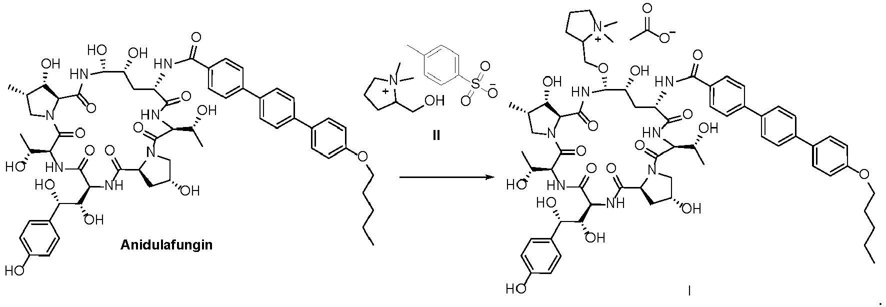

2. The method according to claim 1, wherein the reaction is carried out in the presence of acids A1 and A2; the acid A1 is phenylboronic acid or 3,4-dimethoxyphenylboronic acid, preferably 3,4-dimethoxyphenylboronic acid; the acid A2 is p-toluenesulfonic acid, camphorsulfonic acid or trifluoroacetic acid, preferably camphorsulfonic acid or trifluoroacetic acid, and preferably trifluoroacetic acid.

3. The method according to claim 2, wherein the reaction is carried out in a solvent S1; the solvent S1 is anhydrous dioxane or acetonitrile, preferably acetonitrile.

4. The method according to any one of claims 1-3, wherein the molar ratio of anidulafungin to the compound of formula (II) is 1:(1-50), preferably 1:(1-30), preferably 1:(1-10), preferably 1:(1-6), and preferably 1:30 or 1:6.

5. The method according to any one of claims 2-4, wherein the molar ratio of anidulafungin to the acid A1 is 1:(1-10), preferably 1:(1-5), preferably 1:(1-2), and preferably 1:1.3.

6. The method according to any one of claims 2-5, wherein the molar ratio of anidulafungin to the acid A2 is 1:(1-10), preferably 1:(1-5), and preferably 1:5; or the weight-to-volume ratio of anidulafungin to the acid A2, measured in g/mL, is 1:(1-5), preferably 1:(1-2.5), and preferably 1:2.5.

7. The method according to any one of claims 1-6, wherein the reaction temperature is 0-50 °C, preferably 10-40 °C, preferably 20-30 °C, and preferably room temperature.

8. The method according to any one of claims 1-7, further comprising the following step: reacting N-methylprolinol with methyl p-toluenesulfonate to give a product,

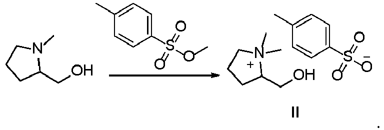

9. The method according to claim 8, wherein the molar ratio of N-methylprolinol to methyl p-toluenesulfonate is 1:(1-5), preferably 1:(1-3), and preferably 1:1.

10. The method according to claim 8 or 9, wherein the reaction for preparing the compound of formula (II) is carried out in a solvent S3, and the solvent S3 is acetone.

11. The method according to any one of claims 8-10, wherein the temperature of the reaction for preparing the compound of formula (II) is 0-80 °C, preferably 10-70 °C, preferably 20-60 °C, preferably 30-60 °C, preferably 40-60 °C, and preferably 50-60 °C.

12. The method according to any one of claims 1-11, wherein the compound of formula (II) is 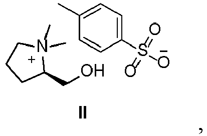 and the compound of formula (I) is

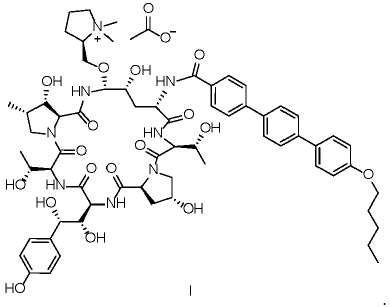

13. An intermediate compound of formula (II):
